(19) Europäisches Patentamt
European Patent Office
Office européen des brevets

(11) Numéro de publication: **0 129 449 B1**

(12) # FASCICULE DE BREVET EUROPEEN

(45) Date de publication du fascicule du brevet: **20.04.88**

(51) Int. Cl.⁴: **C 07 D 213/80**, A 61 K 31/455

(21) Numéro de dépôt: **84400813.6**

(22) Date de dépôt: **20.04.84**

(54) **Diniflumate d'ester morpholinoéthylique d'acide niflumique, préparation, utilisation en thérapeutique comme analgésique et anti-inflammatoire.**

(30) Priorité: **21.04.83 FR 8306579**

(43) Date de publication de la demande:
**27.12.84 Bulletin 84/52**

(45) Mention de la délivrance du brevet:
**20.04.88 Bulletin 88/16**

(84) Etats contractants désignés:
**AT BE CH DE FR IT LI LU NL SE**

(56) Documents cités:
**BE - A - 858 349**
**FR - A - 2 521 997**
**FR - M - 7 963**
**GB - A - 1 146 459**

**Le dossier contient des informations techniques présentées postérieurement au dépôt de la demande et ne figurant pas dans le présent fascicule.**

(73) Titulaire: **HEXACHIMIE Société anonyme dite:, 128, rue Danton, F-92500 Rueil-Malmaison (FR)**

(72) Inventeur: **Bru-Magniez, Nicole, 24, avenue Raphael, F-75016 Paris (FR)**
Inventeur: **Teulon, Jean-Marie, 12 Résidence Bel Ebat 56, avenue de Verdun, F-78170 La Celle-Saint-Cloud (FR)**
Inventeur: **Deghenghi Romano, 11, rue Victor Hugo, F-78230 Le Pecq (FR)**

(74) Mandataire: **Combe, André et al, CABINET BEAU DE LOMENIE 55, rue d'Amsterdam, F-75008 Paris (FR)**

EP 0 129 449 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

**Description**

La présente invention concerne le diniflumate de l'ester β-morpholinoéthylique de l'acide niflumique, de formule I, sa préparation et ses applications thérapeutiques:

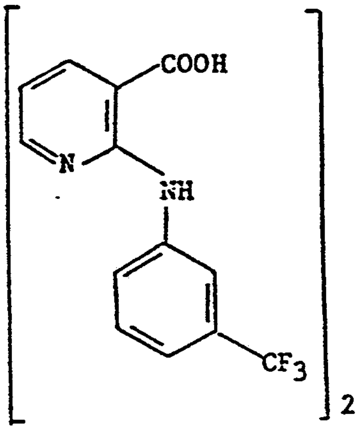

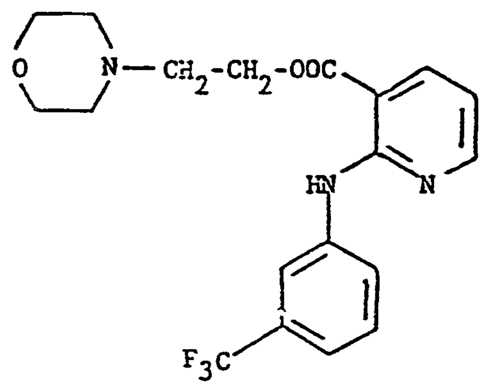

(I)

Ce nouveau produit de formule I selon l'invention est doué d'activités pharmacologiques intéressantes, présentant de nombreux avantages par rapport au médicament connu acide niflumique comme on pourra le constater à la lecture du brevet, et peut être utile en thérapeutique, notamment comme agent analgésique et anti-inflammatoire.

La formation de ce produit bien défini sous forme cristalline stable, ayant un point de fusion bien caractérisé, se fait entre deux molécules d'acide niflumique et une molécule de l'ester β-morpholinoéthylique de l'acide niflumique.

On sait que l'acide niflumique est la DCI de l'acide (trifluorométhyl-3 phénylamino)-2 nicotinique et l'ester aminé est le β-morpholinoéthyle (trifluorométhyl-3 phénylamino)-2 nicotinate (aussi nommé morniflumate).

L'acide niflumique est un médicament bien connu décrit dans le brevet français BSM n° 4.267 M déposé le 09 décembre 1964.

On sait que cet acide niflumique présente des propriétés pharmacologiques remarquables, mais peut induire certains effets secondaires qu'on a tenté d'atténuer en préconisant soit, l'utilisation de certains esters de cet acide (brevet français 7963M) soit, l'utilisation de sels métalliques ou de sels formés d'une mole acide avec une mole d'un dérivé alcoolique de la pyridine (brevet belge 858 349). La présente invention vise à un autre moyen pour permettre l'utilisation des propriétés de l'acide niflumique, ce moyen consistant dans l'emploi d'un sel formé de 2 moles d'acide niflumique pour 1 mole de l'ester β-morpholinoéthylique de cet acide niflumique.

On peut préparer le produit selon l'invention par action du β-morpholinoéthyle (trifluorométhyl-3 phénylamino)-2 nicotinate sur le double de la quantité stoechiométrique de l'acide (trifluorométhyl-3 phénylamino)-2 nicotinique dans un solvant approprié.

On illustre l'invention par les exemples non limitatifs qui suivent.

Exemple 1

A une solution de 99 g de β-morpholinoéthyle (trifluorométhyl-3 phénylamino)-2 nicotinate (0,25 mole) dans 1 l d'éther éthylique, on ajoute une solution de 141 g d'acide (trifluorométhyl-3 phénylamino)-2 nicotinique (0,5 mole) dans 1 l d'éther éthylique. La solution ainsi obtenue est filtrée. L'éther éthylique est évaporé et le résidu obtenu cristallise. En reprenant ces cristaux à chaud dans l'éther isopropylique, puis en refroidissant on obtient après essorage er séchage 218 g de diniflumate d'ester morpholinoéthylique d'acide niflumique sous forme de cristaux jaune pâle de point de fusion (capillaire non corrigé) 109°C. Rendement 91%.

Exemple 2

On dissout dans 175 ml d'acétone un mélange de 19,8 g de β-morpholinoéthyle (trifluorométhyl-3 phénylamino)-2 nicotinate (0,05 mole) et 28,2 g d'acide (trifluorométhyl-3 phénylamino)-2 nicotinique (0,1 mole). La solution est filtrée puis le filtrat est concentré sous vide, le résidu obtenu qui cristallise est repris par 500 ml d'éther isopropylique à chaud. Après refroidissement, les cristaux sont essorés et séchés. On récupère ainsi 45,6 g de diniflumate d'ester morpholinoéthylique d'acide niflumique sous forme de cristaux jaune pâle de point de fusion (capillaire non corrigé) 109°C. Rendement: 95%

Exemple 3

On dissout entre 40 et 45°C dans 175 ml de méthanol un mélange de 19,8 g de β-morpholinoéthyle (trifluorométhyl-3 phénylamino)-2 nicotinate (0,05 mole) et 28,2 g d'acide (trifluorométhyl-3 phénylamino)-2 nicotinique (0,1 mole). Après filtration, la solution est évaporée sous vide et le résidu obtenu cristallise. Les cristaux sont repris à chaud par 250 ml d'éther isopropylique, puis après avoir refroidi on essore les cristaux, puis on les sèche. On obtient ainsi 46 g de diniflumate d'ester morpholinoéthylique d'acide niflumique sous forme de cristaux jaune pâle de point de fusion-

sion (capillaire non corrigé) 109°C. Rendement: 95,8%

Les activités anti-inflammatoire, analgésique et ulcérigène du produit de formule I ont été évaluées parallèlement à celles de l'acide niflumique.

Les résultats montrent que, sur des bases molaires, le produit de formule I est de 2 à 3 fois plus actif que l'acide niflumique sur les tests d'inflammation et d'analgésie. Par contre, le produit de formule I n'est que 1,5 fois plus ulcérigène que l'acide niflumique.

Cet ensemble de résultats est surprenant.

Dans le tableau ci-dessous sont regroupées les doses actives 50 ($DA_{50}$) et ulcérigènes 50 ($DU_{50}$) exprimées en µM/kg VO. L'index pharmacologique répond au rapport:

$$\frac{DA_{50}\ \text{oedème}}{DU_{50}}$$

| | Produit de formule I (invention) | acide niflumique |
|---|---|---|
| Oedème à la carragénie | 50,2 | 111,7 |
| Torsions à la P.B.Q* | 61,0 | 152,4 |
| Action ulcérigène (rats à jeun) | 30,2 | 46,0 |
| Index pharmacologique | 1,66 | 2,43 |

* Phénylbenzoquinone

Les essais ci-dessus ont été menés selon les techniques décrites dans ce qui suit.

Action anti-inflammatoire

Elle a été évalude sur l'oedème à la carragénine.

Technique

Une modification de la méthode de Winter et Coll. (1962) a été utilisée.

Des lots de 12 rats mâles de souche CD (Charles River), pesant 120–150 g, reçoivent pour nourriture et boisson une solution aqueuse à 9% de chlorure de sodium et 15% de glucose, 18 heures avant le test.

Le produit étudié est administré par voie orale en deux fois sous un volume total de 5 ml · 100 $g^{-1}$: une demi-dose 2 heures et une demi-dose 30 minutes avant injection sous-cutanée dans le coussinet plantaire d'une patte postérieure de 0,05 ml d'une solution aqueuse de carragénine à 1,5%. Le volume de la patte est mesuré par pléthysmographie (pléthysmographe Ugo Basile), à intervalles réguliers pendant 4 h 30.

Le tableau ci-dessus donne le pourcentage d'inhibition de la réaction.

Activité analgésique

Antagonisme de la phènylbenzoquinone (torsions à la P.B.Q.)

Technique

L'action analgésique est étudiée par la technique de Siegmund et Coll. (1957).

Des lots de 12 souris mâles, de souche CD 1 (Charles River), pesant 19–22 g reçoivent par voie intrapéritonéale 0,25 ml d'une solution hydro-alcoolique de phénylbenzoquinone à 0,02%, une heure après administration par voie orale du produit étudié (0,5 ml · 20 $g^{-1}$).

On compte le nombre de réactions douloureuses (torsions, étirements) de la 5e à la 10e minute.

Le tableau ci-dessus donne le pourcentage d'inhibition des réactions par rapport aux témoins.

Action ulcérigène

Technique

Des lots de 8 à 14 rats mâles de souche OFA (Iffa Credo) pesant 110–140 g sont mis à la diète hydrique 24 heures avant administration orale du produit étudié sous un volume de 2 ml · 100 $g^{-1}$.

Les animaux sont sacrifiés 6 heures après le traitement; les estomacs sont prélevés, incisés le long de la grande courbure puis cotés de 0 à 3 selon l'échelle décrite par LWOFF (1971):

0 : pas d'ulcère
1 : 1 à 2 ulcères
2 : 3 à 4 ulcères
3 : plus de 4 ulcères

La moyenne des résultats pour un lot, multipliée par le pourcentage de rats présentant des ulcères dans ce lot, donne un index d'ulcération dont le maximum est 300.

Le tableau donne les index d'ulcèration par lot. La dose ulcèrigène 50 ($DU_{50}$) est indiquée en µM/kg.

Conclusions

Les effets anti-inflammatoire et analgèsique du diniflumate de morniflumate, de formule I, ont été évalués vis-à-vis de l'oedème à la carragénine et sur le test des torsions à la phénylbenzoquinone. L'effet ulcérigène a été recherché chez le rat à jeun.

Les résultats obtenus montrent que :

- le diniflumate de morniflumate est 2,2 fois plus actif que l'acide niflumique sur le test d'inflammation aigue;
- son effet analgésique est 2,5 fois supérieur à celui de l'acide niflumique;
- il est 1,5 fois plus ulcérigène que l'acide niflumique.

L'index pharmacologique est nettement supérieur pour le sel de formule I par rapport à l'acide niflumique.

Le produit de formule I pourra être formulé essentiellement en comprimés, gélules, suppositoires, capsules rectales, solutés, gels et pommades à usage thérapeutique, aux doses de 1 à 10 mg/kg de poids corporel.

## Revendications

1. Composé caractérisé en ce qu'il consiste en le diniflumate de l'ester morholinoéthylique de l'acide niflumique, de formule:

(I) 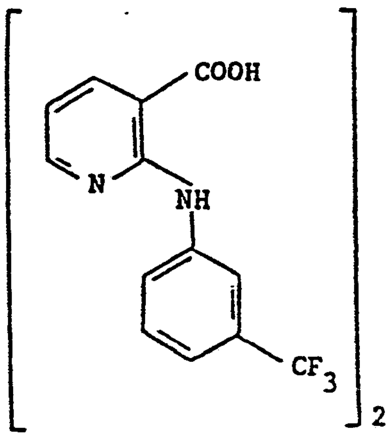

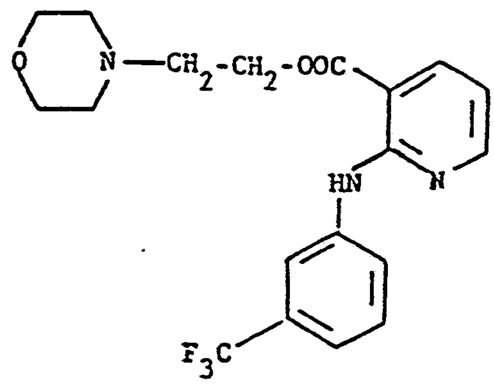

2. Procédé de préparation du produit selon la revendication 1, caractérisé en ce que l'on fait réagir le β-morpholinoéthyle (trifluorométhyl-3 phénylamino)-2 nicotinate sur le double de la quantité stoechiométrique de l'acide (trifluorométhyl-3 phénylamino)-2 nicotinique, dans un solvant approprié comme l'éther éthylique.

3. Médicament analgésique et anti-inflammatoire, caractérisé en ce qu'il contient au moins le composé selon la revendication 1.

## Patentansprüche

1. Verbindung, dadurch gekennzeichnet, dass sie aus dem Diniflumat des Morpholinoäthylesters der Nifluminsäure der Formel

(I) 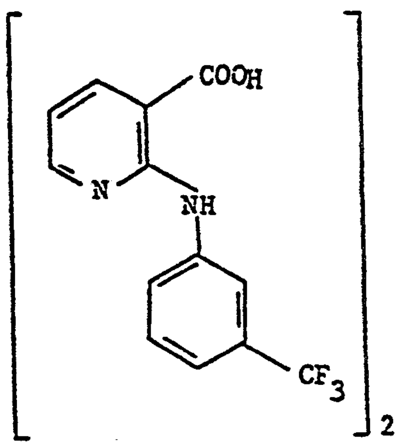

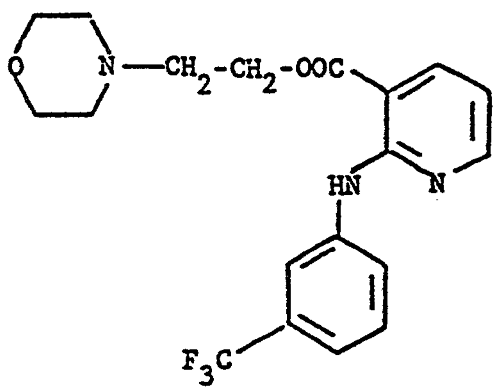

besteht.

2. Verfahren zur Herstellung des Produkts nach Anspruch 1, dadurch gekennzeichnet, dass man β-Morpholinoäthyl-2-(3-trifluormethyl-phenylamino)-nikotinat mit der doppelten stöchiometrischen Menge der 2-(3-Trifluormethyl-phenylamino)-nikotinsäure in einem geeigneten Lösungsmittel, wie Äthyläther, zur Umsetzung bringt.

3. Schmerzstillendes und entzündungshemmendes Medikament, dadurch gekennzeichnet, dass es mindestens die Verbindung nach Anspruch 1 enthält.

## Claims

1. Compound characterized in that it consists in the morpholinoethyl ester diniflumate of niflumic acid, of the formula :

(I) 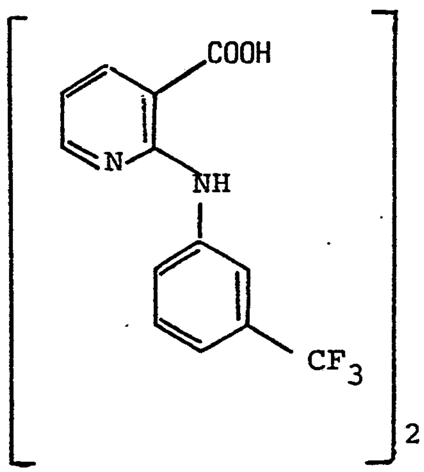

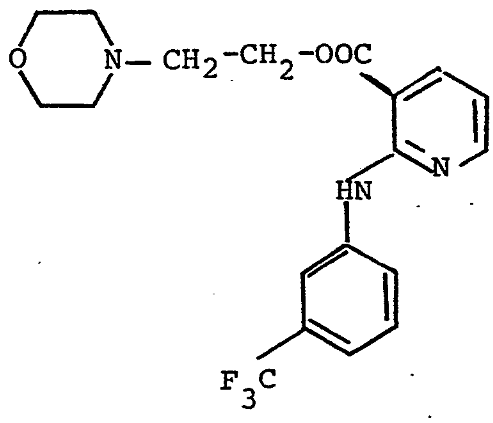

2. Process for preparing the product according to claim 1, characterized in that the β-morpholinoethyl 2-(3-trifluoromethyl phenylamino) nicotinate is reacted on twice the stoichiometric quantity of the 2-(3-trifluoromethyl-phenylamino) nicotinic acid, in an appropriate solvent such as ethyl ether.

3. Analgesic and anti-inflammatory drug, characterized in that it contains at least the compound according to claim 1.